# EUROPEAN PATENT APPLICATION

(11) **EP 4 047 094 A1**
(43) Date of publication of application: **24.08.2022**
(21) Application number: 20877335.8
(22) Date of filing: 15.10.2020
(51) Int. Cl.: C12N 15/113, C07K 14/00, A61K 31/7088, A61P 25/28

(54) **PEPTIDE-NUCLEIC ACID COMPLEX HAVING BLOOD-BRAIN BARRIER PENETRATION ABILITY AND COMPOSITION COMPRISING SAME**

(30) Priority: 16.10.2019 KR 20190128465
(71) Applicant: Seasun Therapeutics, Daejeon 34015 (KR)
(72) Inventor: PARK, Min-Jung, Daejeon 34850 (KR); KIM, Hye Joo, Daejeon 34080 (KR); YU, Ji-Yeon, Cheongju-si Chungcheongbuk-do 28193 (KR); PARK, Hee Kyung, Daejeon 34034 (KR)
(74) Representative: Mewburn Ellis LLP
(86) International application number: PCT/KR2020/014109
(87) International publication number: WO 2021/075880

(57) **Abstract**

The present invention relates to a novel-structured nucleic acid complex having a blood-brain barrier penetration ability and a composition comprising same and, more specifically, to a nucleic acid complex in which a bioactive nucleic acid is complementarily bound to a carrier peptide nucleic acid modified to be generally positively charged, and to a composition for blood-brain barrier penetration comprising same. The nucleic acid complex according to the present invention can penetrate the blood-brain barrier at excellent efficiency, and especially, the conjugation of a drug into the nucleic acid complex increases the blood-brain barrier penetration ability of the drug, and thus is very useful in treatment or diagnosis of diseases.

<110>     SEASUN THERAPEUTICS

<120>     Peptide Nucleic Acid Complex with Blood-Brain Barrier
          Permeability and Composition Comprising the Same

<130>     PP-B2459

<150>     KR 2019-0128465
<151>     2020-10-16

<160>     5

<170>     KoPatentIn 3.0

<210>     1
<211>     15
<212>     DNA
<213>     Artificial Sequence

<220>
<223>     Bioactive Nucleic Acid 1


<400>     1
atgattctgc cctcc                                                          15


<210>     2
<211>     15
<212>     DNA
<213>     Artificial Sequence

<220>
<223>     Carrier Peptide Nucleic Acid 1


<400>     2
tactaagacg ggagg                                                          15


<210>     3
<211>     15
<212>     DNA
<213>     Artificial Sequence

<220>
<223>     Bioactive Nucleic Acid


<400>     3
atgattctgc cctcc                                                          15


<210>     4
<211>     15
<212>     DNA
<213>     Artificial Sequence

<220>
<223>     Carrier Peptide Nucleic Acid


<400>     4

tactaagacg ggagg                                                          15

<210>    5
<211>    13
<212>    PRT
<213>    Artificial Sequence

<220>
<223>    linker peptide

<400>    5
Gly Leu Phe Asp Ile Ile Lys Lys Ile Ala Glu Ser Phe
 1               5                   10

## Description

### Technical Field

The present invention relates to a nucleic acid complex having a novel structure having blood-brain barrier permeability and a composition comprising the same, and more particularly to a nucleic acid complex in which a bioactive nucleic acid and a carrier peptide nucleic acid modified to have an overall positive charge are complementarily bound to each other, and a composition for blood-brain barrier permeation comprising the same.

### Background Art

The blood-brain barrier (BBB) is a structure composed of tight junctions between endothelial cells of the blood vessels in the brain and astrocytes that further strengthen the junctions, and functions to prevent materials in the blood vessels from easily reaching the inside of the brain parenchyma through the blood vessel wall. For this reason, many drugs developed for the treatment of brain diseases have a problem in that they do not cross the blood-brain barrier well. The blood-brain barrier permeation mechanism has not yet been elucidated, and even when the central nervous system is damaged, drugs cannot reach target regions in the central nervous system, and effective treatment methods have not yet been developed.

Recently, in order to promote safer and more effective use of drugs, not only a dosage form suitable for a drug but also a method of efficiently delivering a drug to a site in the body that is the destination for the drug are receiving attention. In particular, there is demand for practical application of a drug delivery system (DDS) that efficiently transports a drug in a required amount to a required place when needed. The development of a carrier that is able to deliver a drug through the blood-brain barrier is also actively underway, but no DDS capable of permeating the blood-brain barrier has been developed.

Meanwhile, unlike traditional drugs, nucleic acid drugs inhibit the expression of target-specific messenger RNA (mRNA), making it possible to address areas of research in which treatment with conventional drugs targeting proteins is impossible (Kole R. et al. Nature Rev. Drug Discov. 2012; 11; 125-140, Wilson C. et al. Curr. Opin. Chem. Bio. 2006; 10: 607-614).

Despite the excellent effects and various applications of gene expression control based on oligonucleic acids, there are many obstacles to be overcome in order to develop therapeutic agents based on nucleic acids. For example, there is a risk of damage to oligonucleic acids by nucleases, etc., and it is impossible to achieve cell membrane permeation by passive diffusion due to the electrical properties (charge) and size of oligonucleic acids. With the goal of solving the above problems, efforts have been made to attain biological stability through modification of nucleic acids, and for modified artificial nucleic acids, it is possible to increase affinity with target nucleic acids without loss of biological activity. A peptide nucleic acid (PNA), which is a kind of modified artificial nucleic acid, has a property of strongly binding to RNA and DNA having a complementary nucleotide sequence, and in particular, is resistant to nucleases and has high biological stability, so research related to therapeutic agents based on various oligonucleic acids is ongoing. However, such a peptide nucleic acid, which is electrically neutral, is disadvantageous in that it is difficult to introduce into a cell (Joergensen M. et al. Oligonucleotides 2011, 21; 29-37). Various clinical trials using nucleic acids are underway due to the performance and advantages thereof when used as drugs, and despite increasing application of nucleic-acid-based therapeutics, the use of carriers for introduction into cells or blood-brain barrier permeation has been extremely limited. In addition, oligonucleic acids alone have very low cell membrane permeability. In particular, since DNA or RNA is negatively charged, it cannot pass through the hydrophobic phospholipid bilayer of the cell, so intracellular delivery through simple diffusion or blood-brain barrier permeation is difficult. In the central nervous system, a method using a viral vector has been proposed (Korean Patent Application Publication No. 2009-159988), but this method has a problem in view of safety and has not been put into practical use.

For this reason, the importance of developing a nucleic acid carrier based on a non-viral oligonucleic acid having low cytotoxicity and low immune activity is continually increasing, and as a result, cell introduction techniques using cationic lipids, liposomes, stable nucleic acid lipid particles (SNALPs), polymers, and cell-penetrating peptides are being developed (Zhi D. et al. Bioconjug. Chem. 2013, 24; 487-519, Buyens K. et al. J. Control Release, 2012, 158; 362-70, ROSSI, J. J. et al. Gene Ther. 2006, 13: 583-584, Yousefi A. et al. J. Control Release, 2013, 170; 209-18, Trabulo S. et al. Curr. Pharm. Des. 2013, 19; 2895-923). Such nucleic acid delivery techniques impart a functional moiety through direct bonding and include a step for forming a complex, but have problems related to endosomal escape efficiency of the liposome structure and biotoxicity. It is necessary to improve the oligonucleic acid introduction function and solve problems related to production procedures and side effects.

In this regard, the present inventors found that the cell permeability of a nucleic acid complex in which a bioactive nucleic acid and a carrier peptide nucleic acid modified to have an overall positive charge are complementarily bound to each other may be greatly improved, and that the expression of a target gene may be very efficiently regulated using the same, and filed a patent for a new nucleic acid construct having low cytotoxicity and improved cell permeation and gene expression control capability of a bioactive nucleic acid (PCT/KR2017/008636).

Accordingly, the present inventors have continuously conducted research on the function of the construct, have endeavored to develop a nucleic acid complex having superior blood-brain barrier permeability, and have ascertained that a nucleic acid complex in which a bioactive nucleic acid and a carrier peptide nucleic acid modified to have an overall positive charge are complementarily bound to each other is able to cross the blood-brain barrier with excellent efficiency, thus culminating in the present invention.

### Summary of the Invention

It is an object of the present invention to provide a nucleic acid complex in which a bioactive nucleic acid and a carrier peptide nucleic acid modified to have an overall positive charge are complementarily bound to each other, a composition for blood-brain barrier permeation comprising the same, and a composition for treating and diagnosing a disease using the same.

It is another object of the present invention to provide a method of treating a disease comprising administering the nucleic acid complex having blood-brain barrier permeability according to the present invention and/or the composition comprising the same to a patient in need of treatment.

In order to accomplish the above objects, the present invention provides a composition for blood-brain barrier permeation comprising a nucleic acid complex having the structure of Structural Formula (1) below:

[Structural Formula (1)] [A ≡ C(+)]

wherein Structural Formula (1),
A represents a bioactive nucleic acid having either a sequence capable of binding to a target gene or a target gene sequence,
C represents a carrier peptide nucleic acid capable of binding to the bioactive nucleic acid;
'≡' represents complementary binding between the bioactive nucleic acid and the carrier peptide nucleic acid,
the bioactive nucleic acid represented by A is a peptide nucleic acid and has an overall negative charge,
the carrier peptide nucleic acid represented by C(+) has an overall positive charge,
the nucleic acid complex of Structural Formula (1) represented by A ≡ C(+) has an overall positive charge, and
the carrier peptide nucleic acid comprises at least one gamma- or alpha-backbone-modified peptide nucleic acid monomer such that the carrier peptide nucleic acid has an overall positive charge, and the gamma- or alpha-backbone-modified peptide nucleic acid monomer comprises monomers having positively charged amino acids, the number of which is higher than the number of monomers having negatively charged amino acids, so the carrier peptide nucleic acid has an overall positive charge.

In addition, the present invention provides a pharmaceutical composition for preventing or treating a disease comprising the nucleic acid complex having the structure of Structural Formula (1).

In addition, the present invention provides a composition or kit for diagnosing a disease comprising the nucleic acid complex having the structure of Structural Formula (1).

In addition, the present invention provides a method of treating a disease comprising administering the nucleic acid complex having the structure of Structural Formula (1) and/or the pharmaceutical composition comprising the same to a patient in need of treatment.

In addition, the present invention provides the use of the nucleic acid complex having the structure of Structural Formula (1) and/or the pharmaceutical composition comprising the same for the prevention or treatment of a disease.

In addition, the present invention provides the application of the nucleic acid complex having the structure of Structural Formula (1) and/or the pharmaceutical composition comprising the same to the manufacture of a medicament for the prevention or treatment of a disease.

In addition, the present invention provides the use of the nucleic acid complex having the structure of Structural Formula (1) and/or the pharmaceutical composition comprising the same for the manufacture of a medicament for the prevention or treatment of a disease.

### Brief Description of Drawings

FIG. 1a shows the results of confocal microscopy analysis on the prefrontal cortex, lateral ventricle, and paraventricular nucleus, which are the anterior regions of the brain tissue, on the 1^{st} day after administration of a nucleic acid complex.
FIG. 1b shows the results of confocal microscopy analysis on the hippocampus, midbrain, and cerebellum, which are the posterior regions of the brain tissue, on the 1^{st} day after administration of the nucleic acid complex.
FIG. 1c shows the results of confocal microscopy analysis on the prefrontal cortex, lateral ventricle, and paraventricular nucleus, which are the anterior regions of the brain tissue, on the 3^{rd} day after administration of the nucleic acid complex.
FIG. 1d shows the results of confocal microscopy analysis on the hippocampus, midbrain, and cerebellum, which are the posterior regions of the brain tissue, on the 3^{rd} day after administration of the nucleic acid complex.
FIG. 2a shows the results of a comparison of distribution of fluorescence expression in each tissue of the brain on the 1^{st} day after administration of the nucleic acid complex.
FIG. 2b shows the results of a comparison of distribution of fluorescence expression in each tissue of the brain on the 3^{rd} day after administration of the nucleic acid complex.
FIG. 3a shows the results of confocal microscopy analysis on the prefrontal cortex, lateral ventricle, and paraventricular nucleus, which are the anterior regions of the brain tissue, on the 1^{st} day after administration of a nucleic acid complex comprising a material for facilitating endosomal escape.
FIG. 3b shows the results of confocal microscopy analysis on the hippocampus, midbrain, and cerebellum, which are the posterior regions of the brain tissue, on the 1^{st} day after administration of the nucleic acid complex comprising the material for facilitating endosomal escape.
FIG. 3c shows the results of confocal microscopy analysis on the prefrontal cortex, lateral ventricle, and paraventricular nucleus, which are the anterior regions of the brain tissue, on the 3^{rd} day after administration of the nucleic acid complex comprising the material for facilitating endosomal escape.
FIG. 3d shows the results of confocal microscopy analysis on the hippocampus, midbrain, and cerebellum, which are the posterior regions of the brain tissue, on the 3^{rd} day after administration of the nucleic acid complex comprising the material for facilitating endosomal escape.
FIG. 4a shows the results of a comparison of distribution of fluorescence expression in each tissue of the brain on the 1^{st} day after administration of the nucleic acid complex comprising the material for facilitating endosomal escape.
FIG. 4b shows the results of a comparison of distribution of fluorescence expression in each tissue of the brain on the 3^{rd} day after administration of the nucleic acid complex comprising the material for facilitating endosomal escape.

### Detailed Description and Preferred Embodiments of the Invention

Unless otherwise defined, all technical and scientific terms used herein have the same meanings as those typically understood by those skilled in the art to which the present invention belongs. In general, the nomenclature used herein is well known in the art and is typical.

An aspect of the present invention pertains to a composition for blood-brain barrier permeation comprising a nucleic acid complex having the structure of Structural Formula (1) below.

[Structural Formula (1)] [A ≡ C(+)]

In Structural Formula (1),
A represents a bioactive nucleic acid having either a sequence capable of binding to a target gene or a target gene sequence,
C represents a carrier peptide nucleic acid capable of binding to the bioactive nucleic acid,
'≡' represents complementary binding between the bioactive nucleic acid and the carrier peptide nucleic acid,
the bioactive nucleic acid represented by A is a peptide nucleic acid and has an overall negative charge,
the carrier peptide nucleic acid represented by C(+) has an overall positive charge,
the nucleic acid complex of Structural Formula (1) represented by A ≡ C(+) has an overall positive charge, and
the carrier peptide nucleic acid comprises at least one gamma- or alpha-backbone-modified peptide nucleic acid monomer such that the carrier peptide nucleic acid has an overall positive charge, and the gamma- or alpha-backbone-modified peptide nucleic acid monomer comprises monomers having positively charged amino acids, the number of which is higher than the number of monomers having negatively charged amino acids, so the carrier peptide nucleic acid has an overall positive charge.

In the present invention, the bioactive nucleic acid or the carrier peptide nucleic acid may comprise a material for facilitating endosomal escape that is additionally bound to the 5'-end or 3'-end of each nucleic acid. Specifically, the nucleic acid complex further comprises a material for facilitating endosomal escape of the bioactive nucleic acid and the carrier peptide nucleic acid, and thus has the structure of Structural Formula (2) below.

Structural Formula (2) [mA ≡ mC(+)]

In Structural Formula (2),
'm' represents a material for facilitating endosomal escape of the bioactive nucleic acid and the carrier peptide nucleic acid.

In the present invention, the "material for facilitating endosomal escape" facilitates escape of the bioactive nucleic acid from the endosome by increasing the osmotic pressure inside the endosome or destabilizing the endosomal membrane. This means that the bioactive nucleic acid moves more efficiently and quickly into the nucleus or cytoplasm to meet and act on a target gene (D.W. Pack, A.S. Hoffman, S. Pun, P.S. Stayton, "Design and development of polymers for gene delivery," Nat. Rev. Drug. Discov., 4, 581-593 (2005)).

In the present invention, the material for facilitating endosomal escape is at least one selected from the group consisting of a peptide, lipid nanoparticles, polyplex nanoparticles, polymer nanospheres, inorganic nanoparticles, cationic lipid-based nanoparticles, a cationic polymer, and a pH-sensitive polymer.

In the present invention, as the material for facilitating endosomal escape, a peptide having the sequence of GLFDIIKKIAESF (SEQ ID NO: 5) may be linked to the bioactive nucleic acid via a linker, and histidine (10) may be linked to the carrier peptide nucleic acid via a linker, but the present invention is not limited thereto.

In the present invention, the lipid nanoparticles may be selected from the group consisting of lipids, phospholipids, cetyl palmitate, Poloxamer 18, Tween 85, tristearin glyceride, and Tween 80.

In the present invention, the polyplex nanoparticles may be poly(amidoamine) or polyethyleneimine (PEI).

In the present invention, the polymer nanospheres may be selected from the group consisting of polycaprolactone, poly(lactide-co-glycolide), polylactide, polyglycolide, poly(d,l-lactide), chitosan, and PLGA-polyethylene glycol.

In the present invention, the inorganic nanoparticles may be selected from the group consisting of Fe₂O₃, Fe₃O₄, WO₃, and WO_{2.9}.

In the present invention, the cationic lipid-based nanoparticles may be selected from the group consisting of 1-(aminoethyl)iminobis[N-(oleicylcysteinyl-1-amino-ethyl)propionamide], N-alkylated derivatives of PTA, and 3,5-didodecyloxybenzamidine.

In the present invention, the cationic polymer may be selected from the group consisting of vinylpyrrolidone-N,N-dimethylaminoethyl methacrylate acid copolymer diethyl sulfate, polyisobutylene, and poly(N-vinylcarbazole).

In the present invention, the pH-sensitive polymer may be selected from the group consisting of polyacids, poly(acrylic acid), poly(methacrylic acid), and hydrolyzed polyacrylamide.

In the present invention, the "bioactive nucleic acid" is a nucleic acid having a complementary sequence capable of binding to a target gene of interest, the expression of which is to be reduced, particularly a complementary sequence capable of binding to mRNA of the target gene of interest, or a nucleic acid comprising a sequence that promotes the expression of a target gene to be expressed. It refers to a nucleic acid involved in gene expression control such as inhibition or promotion of expression of the gene of interest. The bioactive nucleic acid may be a nucleic acid having a sequence complementary to a target gene, the expression of which is to be decreased or increased, or a nucleic acid having a sequence complementary to a single-stranded RNA sequence, such as pre-mRNA, miRNA, mRNA, etc.

In particular, the "bioactive nucleic acid" in the present invention binds to a target gene and a nucleotide sequence comprising the same *in vitro* or *in vivo,* and thus activates or inhibits the intrinsic function of the gene (e.g. transcript expression or protein expression), or regulates splicing of pre-mRNA (e.g. exon skipping). The nucleotide sequence may be a gene regulatory sequence, a gene-coding sequence, or a splicing regulatory sequence. The gene regulatory sequence may be selected from among a promoter, a transcription enhancer, a 5' untranslated region, a 3' untranslated region, a viral packaging sequence, and a selection marker. The gene-coding sequence may be an exon or an intron, and may be located within 10, 5, 3, or 1 kb or 500, 300, or 200 bp from the transcription initiation site of the gene, for example, upstream or downstream of the initiation site. In addition, the splicing regulatory sequence may include a sequence associated with exon skipping, cryptic splicing, pseudo-splice site activation, intron retention, or alternative splicing deregulation.

In the present invention, the "carrier peptide nucleic acid" is a nucleic acid in which some or all bases are complementarily bound to the bioactive nucleic acid to thus impart functionality thereto, and the carrier peptide nucleic acid as used herein may comprise a peptide nucleic acid (PNA) and modified nucleic acids similar thereto, with a peptide nucleic acid being preferred, but the present invention is not limited thereto.

In the present invention, the "nucleic acid complex" enables a bioactive material to penetrate into the body, ultimately into cells, and particularly has the ability to deliver the bioactive material into the body through the blood-brain barrier.

In the present invention, each of the bioactive nucleic acid and the carrier peptide nucleic acid comprises 2 to 50, preferably 5 to 30, more preferably 10 to 25, and most preferably 15 to 17 nucleic acid monomers.

In addition, the bioactive nucleic acid may be composed of a natural nucleobase and/or a modified nucleic acid monomer, and the carrier peptide nucleic acid may be composed of a sequence in which part or all of the nucleotide sequence is complementary to the bioactive nucleic acid.

In particular, the carrier peptide nucleic acid may comprise at least one universal base, and the carrier peptide nucleic acid may be composed exclusively of universal bases.

In the present invention, the bioactive nucleic acid may be selected from the group consisting of DNA, RNA, modified nucleic acids such as PNA (peptide nucleic acid), PMO (phosphorodiamidate morpholino oligonucleotide), LNA (locked nucleic acid), GNA (glycol nucleic acid) and TNA (threose nucleic acid), antisense oligonucleotides, aptamers, siRNA (small interfering RNA), shRNA (short hairpin RNA), ribozymes, and DNAzymes, and the bioactive nucleic acid is preferably selected from the group consisting of DNA, RNA, and modified nucleic acids such as PNA (peptide nucleic acid), PMO (phosphorodiamidate morpholino oligonucleotide), LNA (locked nucleic acid), GNA (glycol nucleic acid) and TNA (threose nucleic acid).

In the present invention, when the monomer used for the bioactive nucleic acid is PNA, it is called a bioactive peptide nucleic acid, and when other monomers are used, they are referred to in the same manner.

In the present invention, the bioactive nucleic acid and the carrier peptide nucleic acid may further comprise at least one functional group selected from the group consisting of phosphodiester, 2'0-methyl, 2'methoxy-ethyl, phosphoramidate, methylphosphonate, and phosphorothioate.

In the present invention, the carrier peptide nucleic acid may be composed of a sequence in which part or all of the nucleotide sequence is complementary to the bioactive nucleic acid. In particular, the carrier peptide nucleic acid may comprise at least one universal base, and the carrier peptide nucleic acid may be composed exclusively of universal bases.

In the present invention, each of the bioactive nucleic acid and the carrier peptide nucleic acid in the nucleic acid complex may have an overall positive charge (cationic), negative charge (anionic), or no charge (neutral), with regard to the electrical properties thereof.

The term "overall" used when representing electrical properties means the general electrical properties of the entire bioactive nucleic acid or carrier peptide nucleic acid from an outside perspective, rather than the electrical properties of individual bases. For example, even though some monomers in the bioactive nucleic acid are positively charged, when the number of negatively charged monomers is larger, the bioactive nucleic acid is negatively charged when considering the "overall" electrical properties, and even though some bases and/or backbones in the carrier peptide nucleic acid are negatively charged, when the number of positively charged bases and/or backbones is larger, the carrier peptide nucleic acid is positively charged when considering the "overall" electrical properties.

Therefore, the nucleic acid complex of the present invention may have an overall positive charge. In the nucleic acid complex, it is preferred that the bioactive nucleic acid be negatively charged when viewing overall electrical properties and that the carrier peptide nucleic acid be positively charged when viewing overall electrical properties, but the present invention is not limited thereto.

In the present invention, the electrical properties of the bioactive nucleic acid and the carrier peptide nucleic acid may be imparted using a modified peptide nucleic acid monomer, and the modified peptide nucleic acid monomer may comprise, as a positively charged carrier peptide nucleic acid, at least one positively charged amino acid selected from the group consisting of lysine (Lys, K), arginine (Arg, R), histidine (His, H), diamino butyric acid (DAB), ornithine (Orn), and amino acid analogues, and may comprise, as a negatively charged carrier peptide nucleic acid, a negatively charged amino acid such as glutamic acid (Glu, E) or a negatively charged amino acid analogue.

In the present invention, the carrier peptide nucleic acid may comprise at least one gamma- or alpha-backbone-modified peptide nucleic acid monomer such that it has an overall positive charge.

The gamma- or alpha-backbone-modified peptide nucleic acid monomer may comprise, in the backbone thereof, at least one positively charged amino acid selected from the group consisting of lysine (Lys, K), arginine (Arg, R), histidine (His, H), diamino butyric acid (DAB), ornithine (Orn), and amino acid analogues in order to attain an electrically positive charge.

In the present invention, modification of the peptide nucleic acid monomer so as to be electrically charged may be performed using a nucleobase-modified peptide nucleic acid monomer, in addition to the backbone modification. Preferably, an amine, triazole, or imidazole moiety is included in the nucleobase in order to attain an electrically positive charge, or carboxylic acid is included in the base in order to attain an electrically negative charge.

In the present invention, the modified peptide nucleic acid monomer of the carrier peptide nucleic acid may further comprise the negative charge in the backbone or nucleobase, but the modified peptide nucleic acid monomer preferably comprises positively charged monomers, the number of which is higher than the number of negatively charged monomers, so the carrier peptide nucleic acid has an overall positive charge.

It is preferred that the nucleic acid complex according to the present invention have an overall positive charge.

In the nucleic acid complex according to the present invention, at least one material selected from the group consisting of a hydrophobic moiety, hydrophilic moiety, target antigen-specific antibody, aptamer, and fluorescent/luminescent label may be bound to the bioactive nucleic acid and/or the carrier peptide nucleic acid, and preferably, at least one material selected from the group consisting of a hydrophobic moiety, hydrophilic moiety, target antigen-specific antibody, aptamer, and fluorescent/luminescent label for imaging is bound to the carrier peptide nucleic acid.

In the present invention, at least one material selected from the group consisting of a hydrophobic moiety, hydrophilic moiety, target antigen-specific antibody, aptamer, quencher, fluorescent label, and luminescent label may be bound to the bioactive nucleic acid and/or the carrier peptide nucleic acid through simple covalent bonding or linker-mediated covalent bonding, but the present invention is not limited thereto. Preferably, the material associated with cell permeability, solubility, stability, transport and imaging (e.g. a hydrophobic moiety, etc.) bound to the nucleic acid carrier is present independently of the bioactive nucleic acid that controls the expression of the target gene.

In the present invention, as described above, the complementary binding form of the bioactive nucleic acid and the carrier peptide nucleic acid is largely based on antiparallel binding or parallel binding. The complementary binding form is configured such that the bioactive nucleic acid is released in the presence of a target sequence (a sequence complementary to the bioactive nucleic acid).

The antiparallel binding and the parallel binding are determined depending on the 5' orientation and the 3' orientation in the DNA-DNA or DNA-PNA binding mode. Antiparallel binding is a typical method for DNA-DNA or DNA-PNA binding. Taking the nucleic acid complex according to the present invention as an example, the bioactive nucleic acid in the 5' to 3' orientation and the carrier peptide nucleic acid in the 3' to 5' orientation are bound to each other. Parallel binding is a binding form in which binding affinity is somewhat lower than that of antiparallel binding, and both the bioactive nucleic acid and the carrier peptide nucleic acid are bound to each other in the 5' to 3' orientation or the 3' to 5' orientation.

In the nucleic acid complex according to the present invention, it is preferred that the binding affinity between the bioactive nucleic acid and the carrier peptide nucleic acid be lower than the binding affinity between the bioactive nucleic acid and a gene targeted by the bioactive nucleic acid, particularly mRNA of the target gene. The binding affinity is determined based on a melting temperature Tm.

An example of a specific method for making the binding affinity (melting temperature, Tm) between the bioactive nucleic acid and the carrier peptide nucleic acid lower than the binding affinity between the bioactive nucleic acid and the gene targeted by the bioactive nucleic acid, particularly mRNA of the target gene, may comprise parallel binding or partial specific binding of the bioactive nucleic acid and the carrier peptide nucleic acid, but the present invention is not limited thereto.

In another example, the carrier peptide nucleic acid may comprise at least one peptide nucleobase selected from the group consisting of a linker, a universal base, and a peptide nucleobase having a base that is not complementary to the corresponding base of the bioactive nucleic acid, but the present invention is not limited thereto.

In the present invention, the universal base is a base pairing with a natural base such as adenine, guanine, cytosine, thymine, or uracil without selectivity and having binding affinity lower than the complementary binding affinity, and is at least one selected from the group consisting of inosine PNA, indole PNA, nitroindole PNA, and abasic PNA, preferably inosine PNA.

In the present invention, a combination of the binding form and the electrical properties of nucleic acids for controlling the function of the nucleic acid complex is provided, and the combination of the binding form and the electrical properties of nucleic acids enables the particle size and the time of action to be controlled and cell permeability, solubility and specificity to be improved.

In the present invention, it is possible to control the time point at which the bioactive peptide nucleic acid is bound to the target sequence in the presence of the target gene by adjusting the binding affinity between the bioactive peptide nucleic acid and the carrier peptide nucleic acid (the time point of displacement of the strand of the bioactive nucleic acid to the target sequence, and the time point of target-specific release and binding of the bioactive nucleic acid).

In the nucleic acid complex according to the present invention, the time point of displacement of the strand of the bioactive nucleic acid to the target gene and the time point of target-specific release and binding of the bioactive nucleic acid may be controlled by varying the presence, number, and position of non-specific bases, universal bases, and linkers of the carrier peptide nucleic acid for non-specific binding of the complex. Control may be performed through a combination of the above conditions and the complementary binding form of the peptide complex, such as parallel binding or antiparallel binding.

Another aspect of the present invention pertains to a composition for diagnosing or treating a disease comprising a nucleic acid complex having the structure of Structural Formula (1) below and having blood-brain barrier permeability.

[Structural Formula (1)] [A ≡ C(+)]

In Structural Formula (1),
A represents a bioactive nucleic acid having either a sequence capable of binding to a target gene or a target gene sequence,
C represents a carrier peptide nucleic acid capable of binding to the bioactive nucleic acid;
'≡' represents complementary binding between the bioactive nucleic acid and the carrier peptide nucleic acid,
the bioactive nucleic acid represented by A is a peptide nucleic acid and has an overall negative charge,
the carrier peptide nucleic acid represented by C(+) has an overall positive charge,
the nucleic acid complex of Structural Formula (1) represented by A ≡ C(+) has an overall positive charge, and
the carrier peptide nucleic acid comprises at least one gamma- or alpha-backbone-modified peptide nucleic acid monomer such that the carrier peptide nucleic acid has an overall positive charge, and the gamma- or alpha-backbone-modified peptide nucleic acid monomer comprises monomers having positively charged amino acids, the number of which is higher than the number of monomers having negatively charged amino acids, so the carrier peptide nucleic acid has an overall positive charge.

As used herein, the terms "blood-brain barrier" and "BBB" are used interchangeably, and are used to refer to a permeable barrier that exists in blood vessels as they travel through the brain tissue that closely regulates and severely restricts exchange between the blood and the brain tissue. Components of the blood-brain barrier include the endothelial cells, which form the deepest lining of all blood vessels, the tight junctions between adjacent endothelial cells that are structurally correlated with BBB, the basement membrane of endothelial cells, and the expanded foot processes of nearby astrocytes, covering almost all of the exposed outer surface of blood vessels. The BBB prevents most materials in the blood, for example most large molecules, such as Ig, antibodies, complement, albumin, and drugs, as well as small molecules, from entering the brain tissue.

As used herein, the terms "disease" and "disorder" are used interchangeably, and refer to any change in a physical condition or some organ that impedes or disrupts the performance of a function and/or causes symptoms such as discomfort, dysfunction, distress, and the like, or even kills an afflicted person or causes death of someone in contact therewith.

In the present invention, the bioactive nucleic acid may comprise a material for treating or diagnosing a disease that is additionally bound to the 5'-end or 3'-end of the nucleic acid.

In the present invention, the additionally bound material may comprise an active agent. The active agent may be a material that is known not to cross the blood-brain barrier, or it may be a material not capable of permeating the same. Moreover, the active agent may be a material that permeates the blood-brain barrier only in small amounts and thus cannot be delivered in an effective amount without the presence of permeation aids.

The active agent may be a pharmaceutically active agent, a diagnostically active agent, or a combination thereof.

The material for treating a brain disease that may be linked to the bioactive nucleic acid of the present invention may be used without limitation, so long as it is conventionally used for the treatment of such a disease. It may include, for example, a neuroprotective agent, such as NMDA (N-methyl-d-aspartate) receptor inhibitor, acetylcholinesterase inhibitor, anti-amyloid protein agent, etc., examples of which may include donepezil, galantamine, tacrine, memantine, etc. In addition, it may be, for example, temozolomide, carmustine-containing Gliadel wafer, melphalan, pregabalin, Depakote, or methotrexate.

In addition, the material for treating a brain disease that may be linked to the bioactive nucleic acid of the present invention may be used without limitation, so long as it is conventionally used for the treatment of brain tumors, and among existing anticancer drugs, doxorubicin, paclitaxel, etc., which have difficulty crossing the blood-brain barrier, may be linked to the bioactive nucleic acid and used to treat brain tumors.

The linking of the agent and the bioactive nucleic acid of the present invention may be performed through a method known in the art, for example, covalent bonding or linker-mediated covalent bonding. To this end, as necessary, the bioactive nucleic acid of the present invention may be chemically modified within a range within which the activity thereof is not lost.

Still another aspect of the present invention pertains to a composition or kit for diagnosing a brain disease comprising the nucleic acid complex having the structure of Structural Formula (1) and having blood-brain barrier permeability.

In the present invention, the material for diagnosing a brain disease that may be additionally linked to the nucleic acid complex may comprise a contrast agent, preferably a vascular contrast agent that may be administered intravenously, for example, an iodide contrast agent or a gadolinium contrast agent. In particular, it is more preferably a radiotracer for brain positron emission tomography (PET) such as [11C] 25B-NBOMe, [18F] altanserin, [11C] carfentanil, [11C] DASB, [11C] DTBZ or [18F] fluoropropyl-DTBZ, [11C] ME@HAPTHI, [18F] fallypride, [18F] florbetaben, [18F] florbetapir, [18F] or [11C] flumazenil, [18F] flutemetamol, [18F] fluorodopa, [18F] desmethoxyfallypride, [18F] mefway, [18F] MPPF, [18F] nifene, [11C] raclopride, [18F] setoperone, [18F] or [11C] N-methylspiperone, [11C] verapamil, Pittsburgh compound B, 18F-THK5105, 18F-THK5117, and 18F-THK5351.

Yet another aspect of the present invention pertains to a pharmaceutical composition for preventing or treating a brain disease comprising the nucleic acid complex having the structure of Structural Formula (1) and having blood-brain barrier permeability.

Still yet another aspect of the present invention pertains to a method of preventing or treating a brain disease comprising administering the nucleic acid complex having the structure of Structural Formula (1) and having blood-brain barrier permeability to a subject.

A further aspect of the present invention pertains to the use of the nucleic acid complex having the structure of Structural Formula (1) and having blood-brain barrier permeability for the manufacture of a medicament for the prevention or treatment of a brain disease.

In the present invention, the disease is preferably a brain disease, but is not limited thereto.

In the present invention, the brain disease is preferably selected from the group consisting of Alzheimer's disease, Parkinson's disease, Huntington's disease, Lou Gehrig's disease, Creutzfeldt-Jakob disease, Niemann-Pick disease, stroke, palsy, dementia, thrombosis, embolism, transient ischemic attack, small artery occlusion, intracerebral hemorrhage, cerebral infarction, head injury, cerebral circulation metabolic disorder, brain functional coma, brain cancer, and brain tumor, but is not limited thereto.

In the present invention, the term "composition for treatment" may be used interchangeably with "pharmaceutical composition", and the composition comprises, as an active ingredient, the nucleic acid complex of the present invention comprising the bioactive nucleic acid and the carrier peptide nucleic acid binding to the nucleic acid, and may take a form in which a therapeutic drug for treating a target disease is additionally bound to the nucleic acid complex.

The composition for treatment according to the present invention may be formulated in a dosage form to be delivered through the blood-brain barrier according to standard pharmaceutical practice. These formulations may comprise additives such as carriers, excipients, adjuvants or diluents suitable for a formulation in a pharmaceutically acceptable form, in addition to the active ingredient.

The term "pharmaceutically acceptable" refers to properties that do not impair the biological activity or properties of a compound.

The term "carrier" is defined as a compound that facilitates the addition of a nucleic acid complex into a cell or tissue. For example, dimethylsulfoxide (DMSO) is a commonly used carrier that facilitates the introduction of many organic compounds into cells or tissues of living organisms.

The term "diluent" is defined as a compound that stabilizes the biologically active form of the compound of interest and is also diluted in water which dissolves the compound. A salt dissolved in a buffer solution is used as a diluent in the art. A commonly used buffer solution is phosphate-buffered saline, because it mimics the salinity of human fluids. Since buffer salts may control the pH of a solution at low concentrations, buffer diluents rarely modify the biological activity of a compound.

The material comprising the nucleic acid complex according to the present invention may be administered to a patient as a pharmaceutical composition alone or in combination with other active ingredients, as in combination therapy, or with suitable carriers or excipients.

A pharmaceutical composition suitable for use in the present invention comprises a composition in which the active ingredients are contained in amounts effective to achieve the intended purposes thereof. More specifically, a therapeutically effective amount is an amount of a compound that is effective to prolong the lifetime of the subject to be treated, or to prevent, alleviate, or reduce symptoms of a disease. The determination of a therapeutically effective amount is within the capability of those skilled in the art, particularly in light of the detailed disclosure provided herein.

As used herein, the term "prevention" refers to any action that prevents the onset of a disease or inhibits progression thereof through administration of the composition for treatment comprising the nucleic acid complex.

As used herein, the term "amelioration" refers to any action of at least reducing a parameter related to the status of a disease to be treated, for example, the extent of symptoms, through administration of the composition for treatment comprising the nucleic acid complex.

As used herein, the term "treatment" refers to any action by which symptoms of a disease are ameliorated or eliminated through administration of the composition for treatment comprising the nucleic acid complex.

The composition comprising the nucleic acid complex according to the present invention may be applied in a pharmaceutically effective amount in order to treat a brain disease or to suppress (or alleviate) symptoms of a brain disease. The pharmaceutically effective amount may vary depending on various factors, such as the type of brain disease, the age and body weight of the patient, characteristics and extent of symptoms, therapy currently being administered, the number of types of therapy, the form and route of application, etc., and may be easily determined by experts in the field. The composition of the present invention may be applied together or sequentially with the aforementioned pharmacological or physiological components, and may also be applied in combination with additional conventional therapeutic agents, or may be applied sequentially or simultaneously with conventional therapeutic agents. These applications may be carried out one or multiple times.

In the present invention, the "subject" means a mammal, preferably a human, suffering from or at risk of developing a condition or disease that may be alleviated, suppressed or treated by administering the nucleic acid complex according to the present invention.

In addition, the dose of the compound of the present invention for a human body may vary depending on the patient's age, body weight, and gender, dosage form, health status, and the severity of disease, and based on an adult patient weighing 70 kg, the compound may typically be administered in a dose of 0.001 to 1,000 mg/day, and preferably 0.01 to 500 mg/day, and may be administered once a day or several times a day at regular time intervals according to the judgment of a doctor or pharmacist.

The toxicity and therapeutic efficacy of the composition comprising the nucleic acid complex as described herein may be estimated by standard pharmaceutical procedures in cell culture or experimental animals in order to determine, for example, an LD50 (a dose lethal to 50% of the population), an ED50 (a dose having a therapeutic effect on 50% of the population), or an IC50 (a dose having a therapeutic inhibitory effect on 50% of the population). The dose ratio between the toxicity and the therapeutic effect is a therapeutic index, which may be represented as the ratio between LD50 and ED50 (or IC50). Compounds exhibiting high therapeutic indices are preferred. Data obtained from cell culture assays may be used to estimate a range of doses for use in humans. The dosages of these compounds preferably fall within a range of circulating concentrations that include ED50 (or IC50) with little or no toxicity.

As used herein, the term "administering" or "administration" refers to the action of introducing the pharmaceutical composition of the present invention to a subject through any suitable method, and the route of administration may be varied, and may be either oral or parenteral, so long as it is able to reach the target tissue.

The pharmaceutical composition of the present invention may be administered in a pharmaceutically effective amount. In the present invention, the term "pharmaceutically effective amount" is an amount sufficient to treat or prevent a disease at a reasonable benefit/risk ratio applicable to medical treatment or prevention, and the effective dose level may be determined depending on factors including the severity of disease, drug activity, patient's age, body weight, health, gender, and drug sensitivity, administration time of the composition of the present invention, administration route, excretion rate, duration of treatment, and drugs used in combination or simultaneously with the composition of the present invention, and other factors well known in the medical field.

The pharmaceutical composition according to the present invention may be administered as an individual therapeutic agent or in combination with other therapeutic agents, may be administered sequentially or simultaneously with conventional therapeutic agents, and may be administered one or multiple times. Taking into consideration all of the above factors, it is important to administer the composition in the minimum amount capable of obtaining the maximum effect without side effects.

The dose of the pharmaceutical composition of the present invention may be determined by those skilled in the art in consideration of the purpose of use, the severity of disease, the patient's age, body weight, gender, and disease history, or the type of material used as an active ingredient. For example, the pharmaceutical composition of the present invention may be administered to mammals including humans in an amount of 10 to 100 mg/kg, and preferably 10 to 30 mg/kg, in one day. The dose of the composition of the present invention may be divided into multiple doses to realize an administration frequency of 1 to 3 times a day or several times a day, but is not particularly limited thereto.

Hereinafter, the present invention will be described in more detail with reference to examples. It will be obvious to those skilled in the art that these examples are merely to illustrate the present invention, and the scope of the present invention is not limited by these examples.

### Example 1: Bioactive nucleic acid, carrier peptide nucleic acid, and production of complex using the same

In the present invention, in order to evaluate the effect of the nucleic acid complex on blood-brain barrier permeability, a complex of a bioactive nucleic acid (antisense PNA) in which vascular endothelial growth factor (VEGF) antisense nucleic acid (antisense PNA) was labeled with methotrexate (MTX) and a fluorescent material (cy3) and a carrier nucleic acid (carrier PNA) was used. Methotrexate (MTX) is known to cross the blood-brain barrier, but it is a brain disease therapeutic agent having very low bioavailability. Methotrexate has low bioavailability but enables blood-brain barrier permeation and is thus used as a therapeutic agent for brain tumors, making it possible to evaluate the effect of increasing the blood-brain barrier permeability thereof owing to the nucleic acid complex. In a positive control group, a synthetic material in which methotrexate was labeled only with a fluorescent material was used.

The nucleotide sequences, monomer modifications and structures of the PNA-based bioactive nucleic acid and carrier peptide nucleic acid used in this Example are shown in Table 1 below.

All peptide nucleic acids used in the present invention were synthesized through HPLC purification by Panagene (Korea).

The sequence information of the bioactive peptide nucleic acid and the carrier peptide nucleic acid used in the present invention is shown in Table 1 below.

**[Table 1]**

| Sequences of bioactive nucleic acid and carrier peptide nucleic acid for confirming blood-brain barrier permeability | | | |
|---|---|---|---|
| Classification | SEQ ID NO | Nucleotide sequence | Monomer modification |
| Bioactive nucleic acid | SEQ ID NO: 1 | 5'-compound-OAT⁽⁻⁾GA⁽⁺⁾TTC⁽⁻⁾TG⁽⁺⁾CCC⁽⁻⁾TCC-O-K(cy3)-3' | -+-+- |
| Carrier peptide nucleic acid | SEQ ID NO: 2 | 5'-TAC⁽⁺⁾TAAGA⁽⁺⁾CGG⁽⁺⁾GAGG-O-K-3' | +++ |
| Methotrexate | | compound-O-K(cy3) | |

| | | | |
|---|---|---|---|
| (Compound = MTX) | | | |

Monomer modification for imparting electrical properties was carried out such that a modified peptide backbone was constructed by including, in the backbone of the peptide nucleic acid, lysine (Lys, K, (+)) in order to attain an electrically positive charge and glutamic acid (Glu, E, (-)) in order to attain an electrically negative charge.

The bioactive nucleic acid and the carrier peptide nucleic acid were hybridized under DMSO, and as a result, a complex composed of the bioactive nucleic acid and the carrier peptide nucleic acid was constructed.

### Example 2: Analysis of blood-brain barrier permeability using nucleic acid complex

The blood-brain barrier permeability of the nucleic acid complex was analyzed in a mouse model using the nucleic acid complex constructed so as to have the structure shown in Table 2 below according to Example 1.

### Example 2-1: Administration of complex of bioactive peptide nucleic acid and carrier peptide nucleic acid to animal model

In order to confirm the blood-brain barrier permeability of the nucleic acid complex, 6-week-old male ICR mice (OrientBio, Korea) were purchased and acclimatized for a week, after which 20 mg/kg (n=6) of the nucleic acid complex was administered to the caudal veins thereof. In a positive control group (n=6), fluorescent-material-labeled methotrexate was administered to the caudal veins, and in a negative control group (n=6), DMSO at the same concentration as in the experimental group was diluted with saline and administered via the same route.

The nucleic acid complex combination used in this Example is shown in Table 2 below.

**[Table 2]**

| Nucleic acid complex combination for confirming blood-brain barrier permeation in animal model | |
|---|---|
| Classification | Nucleic acid complex |
| POLIGO^{™}_1 | SEQ ID NOS: 1 and 2 |

### Example 2-2: Separation and analysis of brain tissue in animal model

The experiment was carried out under the conditions of Example 2-1, and the tissues in the experimental group and the control group were separately observed on the 1^{st} day and the 3^{rd} day. The mouse model was anesthetized by intraperitoneal administration of Avertin (5 mg/kg), followed by washing and fixing of the brain tissue through perfusion with saline and 4% paraformaldehyde. The fixed brain tissue was separated and dehydrated by placing the same in 30% sugar water for 24 hours, after which an embedding process was performed using OCT. The entire region of the brain tissue was cryosectioned into 30 µm sections. In order to observe the entire brain tissue from the cerebral cortex to the brainstem, the tissues were broadly separated into a total of six regions, namely the prefrontal cortex, lateral ventricle, paraventricular nucleus, hippocampus, midbrain, and cerebellum, and mounted on slide glasses, and the nuclei were stained with DAPI for 15 minutes and then analyzed using a confocal microscope.

When a drug is administered intravenously, it is common for the circulation path thereof into the brain tissue through the blood to spread from the back part of the brain toward the front part over time. Therefore, in order to determine whether the administered material spreads through the present system, the brain tissue was classified into six major regions from the front to the back and analyzed. In addition, in order to observe the extent of spreading over time, the groups were separately observed on day 1 and day 3, and the numerical values thereof were analyzed.

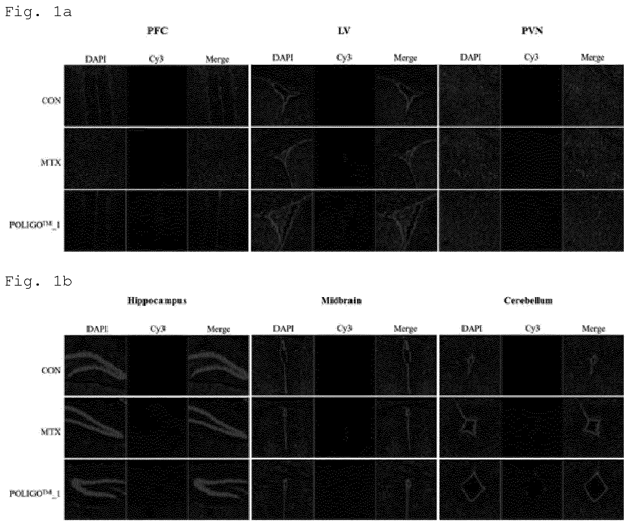

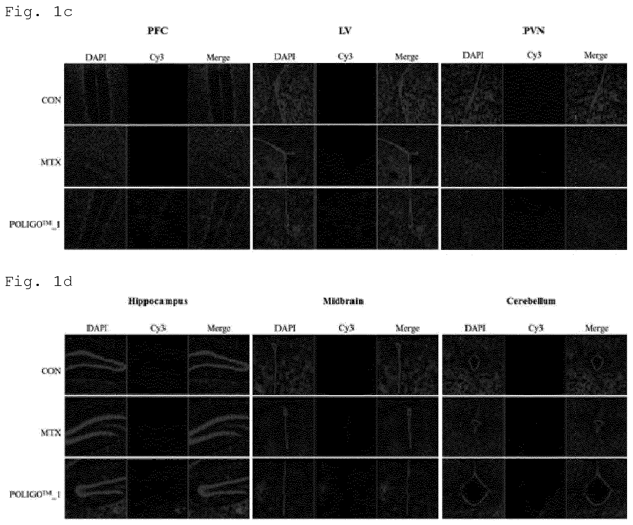

As shown in FIG. 1, it was confirmed that fluorescence expression by cy3 appeared in the experimental group administered with the nucleic acid complex, which showed high fluorescence expression compared to the group administered only with methotrexate, which is known to cross the blood-brain barrier. Therefore, it was confirmed that the blood-brain barrier permeability and bioavailability of methotrexate were increased owing to the nucleic acid complex.

When the groups were separately observed on day 1 and day 3 and the numerical values thereof were analyzed, on day 1, the fluorescence value of the nucleic acid complex was determined to be higher in the midbrain and cerebellum, which are the posterior regions of the brain tissue (FIG. 1b), than in the prefrontal cortex and paraventricular nucleus, which are the anterior regions thereof (FIG. 1a), and on day 3, it was confirmed that the fluorescence spread to the prefrontal cortex, which is the anterior region of the brain tissue, and thus the fluorescence expression was high (FIGS. 1c and 1d). Therefore, it was confirmed that the nucleic acid complex permeated and spread along the circulatory system of blood vessels and brain tissue regions.

Then, for all experimental groups to which the nucleic acid complex was administered, the distribution of fluorescence in tissues depending on the administration date was numerically analyzed, and the distribution of fluorescence expression in each tissue of the brain after administration of the nucleic acid complex was compared.

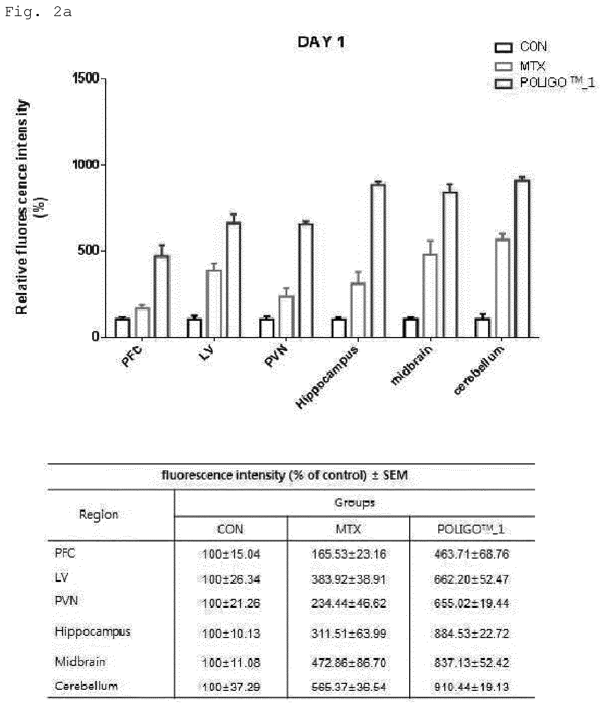

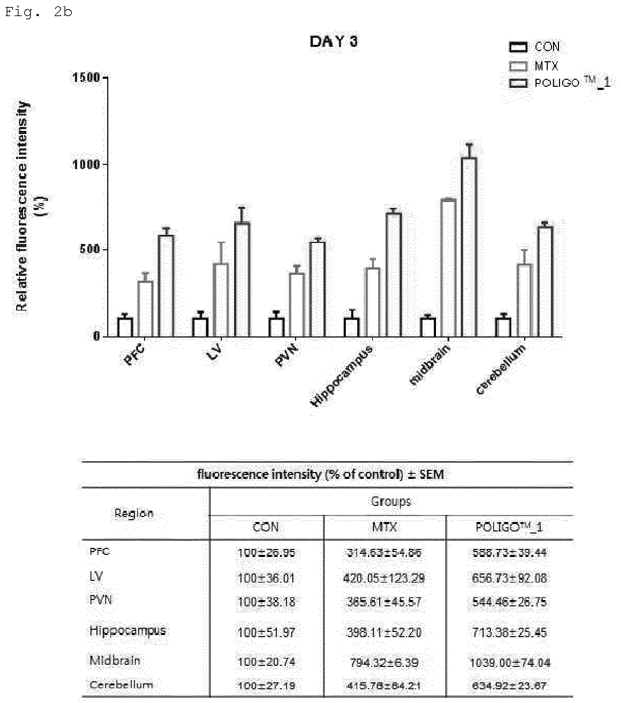

As a result, on day 1, distribution of fluorescence expression of the nucleic acid complex was determined to be high in the midbrain and cerebellum, which are the posterior regions of the brain, among various brain regions (FIG. 2a). The fluorescence value was found to have increased about 2 to 3 times in the entire brain in the group administered with the nucleic acid complex compared to the positive control group administered with MTX alone. On day 3, the distribution of fluorescence expression was determined to be high even in the prefrontal cortex, which is the anterior region of the brain, among the brain regions (FIG. 2b). Like the results of day 1, it was confirmed that the fluorescence value of the group administered with the nucleic acid complex was about 1.5 to 2 times as high as that of the positive control group administered with MTX alone. In addition, it was confirmed that fluorescence expression was higher on day 3 than on day 1 in all brain tissue regions. By numerically analyzing the fluorescence intensity, the accuracy of the experimental results was increased.

### Example 3: Bioactive peptide nucleic acid and carrier peptide nucleic acid comprising material for facilitating endosomal escape, and production of complex using the same

In the present invention, in order to evaluate the effect of the nucleic acid complex on blood-brain barrier permeability, a complex of a bioactive nucleic acid (antisense PNA) comprising a material for facilitating endosomal escape in which a vascular endothelial growth factor (VEGF) antisense nucleic acid (antisense PNA) was labeled with chlorambucil and a fluorescent material (cy3) and a carrier nucleic acid (carrier PNA) comprising a material for facilitating endosomal escape was constructed and used. Chlorambucil is a drug known to be incapable of crossing the blood-brain barrier.

The nucleotide sequences, monomer modifications and structures of the PNA-based bioactive nucleic acid and carrier peptide nucleic acid used in this Example are shown in Table 3 below.

All peptide nucleic acids used in the present invention were synthesized through HPLC purification by Panagene (Korea).

The sequence information of the bioactive peptide nucleic acid and the carrier peptide nucleic acid used in the present invention is shown in Table 3 below.

**[Table 3]**

| Sequences of bioactive nucleic acid and carrier peptide nucleic acid comprising material for facilitating endosomal escape for confirming blood-brain barrier permeability | | | |
|---|---|---|---|
| Classification | SEQ ID NO | Nucleotide sequence | Monomer modification |
| Bioactive nucleic acid | SEQ ID NO: 3 | 5'-compound-O-GLFDIIKKIAESF-O-AT⁽⁻⁾GA⁽⁺⁾TTC⁽⁻⁾TG⁽⁺⁾CCC⁽⁻⁾TCC-O-K(cy3)-3' | -+-+- |
| Carrier peptide nucleic acid | SEQ ID NO: 4 | 5'-Histidine(10)-O-TAC⁽⁺⁾TAAGA⁽⁺⁾CGG⁽⁺⁾GAGG-O-K-3' | +++ |

| | | | |
|---|---|---|---|
| (Compound = chlorambucil) | | | |

Monomer modification for imparting electrical properties was carried out such that a modified peptide backbone was constructed by including, in the backbone of the peptide nucleic acid, lysine (Lys, K, (+)) in order to attain an electrically positive charge and glutamic acid (Glu, E, (-)) in order to attain an electrically negative charge.

The bioactive nucleic acid and the carrier peptide nucleic acid were hybridized under DMSO, and as a result, a complex composed of the bioactive nucleic acid and the carrier peptide nucleic acid was constructed.

### Example 4: Analysis of blood-brain barrier permeability using nucleic acid complex comprising material for facilitating endosomal escape

The blood-brain barrier permeability of the nucleic acid complex was analyzed in a mouse model using the nucleic acid complex constructed so as to have the structure shown in Table 4 below according to Example 3.

### Example 4-1: Administration of complex of bioactive peptide nucleic acid and carrier peptide nucleic acid to animal model

In order to confirm the blood-brain barrier permeability of the nucleic acid complex comprising a material for facilitating endosomal escape, 6-week-old male ICR mice (OrientBio, Korea) were purchased and acclimatized for a week, after which 20 mg/kg (n=6) of the nucleic acid complex was administered to the caudal veins thereof. In a control group (n=2), DMSO at the same concentration as in the experimental group was diluted with saline and administered to the caudal veins.

The nucleic acid complex combination used in this Example is shown in Table 4 below.

**[Table 4]**

| Nucleic acid complex combination comprising material for facilitating endosomal escape for confirming blood-brain barrier permeation in animal model | |
|---|---|
| Classification | Nucleic acid complex |
| POLIGO^{™}_2 | SEQ ID NOS: 3 and 4 |

### Example 4-2: Separation and analysis of brain tissue in animal model

The experiment was carried out under the conditions of Example 4-1, and the tissues in the experimental group and the control group were separately observed on the 1^{st} day and the 3^{rd} day. The mouse model was anesthetized by intraperitoneal administration of Avertin (5 mg/kg), followed by washing and fixing of the brain tissue through perfusion with saline and 4% paraformaldehyde. The fixed brain tissue was separated and dehydrated by placing the same in 30% sugar water for 24 hours, after which the embedding process was performed using OCT. The entire region of the brain tissue was cryosectioned into 30 µm sections. In order to observe the entire brain tissue from the cerebral cortex to the brainstem, the tissues were broadly separated into a total of six regions, namely the prefrontal cortex, lateral ventricle, paraventricular nucleus, hippocampus, midbrain, and cerebellum, and mounted on slide glasses, and the nuclei were stained with DAPI for 15 minutes and then analyzed using a confocal microscope.

When a drug is administered intravenously, it is common for the circulation path thereof into the brain tissue through the blood to spread from the back part of the brain toward the front part over time. Therefore, in order to determine whether the administered material spreads through the present system, the brain tissue was classified into six major regions from the front to the back and analyzed. In addition, in order to observe the extent of spreading over time, the groups were separately observed on day 1 and day 3, and the numerical values thereof were analyzed.

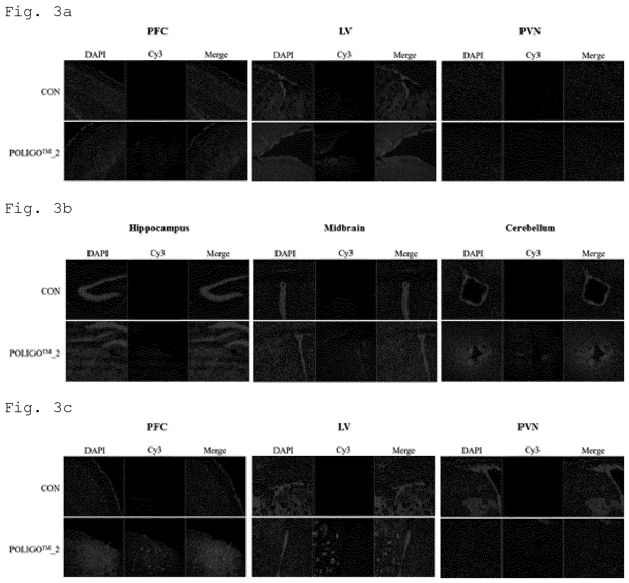

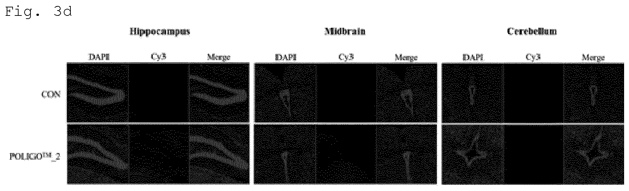

As shown in FIG. 3, it was confirmed that fluorescence expression by cy3 appeared in the experimental group administered with the nucleic acid complex comprising the material for facilitating endosomal escape, indicating that the conventional drug, which is incapable of crossing the blood-brain barrier, successfully permeated the brain owing to the nucleic acid complex, despite labeling.

In addition, when the groups were separately observed on day 1 and day 3 and the numerical values thereof were analyzed, on day 1, the fluorescence value of the nucleic acid complex was determined to be higher in the midbrain and cerebellum, which are the posterior regions of the brain tissue (FIG. 3b), than in the prefrontal cortex and lateral ventricle, which are the anterior regions thereof (FIG. 3a). On day 3, the fluorescence value was determined to be high even in the prefrontal cortex, which is the anterior region (FIG. 3c), but the fluorescence value was remarkably decreased in the midbrain and cerebellum, which are the posterior regions of the brain, compared to day 1 (FIG. 3d). Thereby, it was confirmed that the nucleic acid complex permeated and spread along the circulatory system of blood vessels and brain tissue regions.

Then, for all experimental groups to which the nucleic acid complex was administered, the distribution of fluorescence in tissues depending on the administration date was numerically analyzed, and distribution of fluorescence expression in each tissue of the brain after administration of the nucleic acid complex was compared.

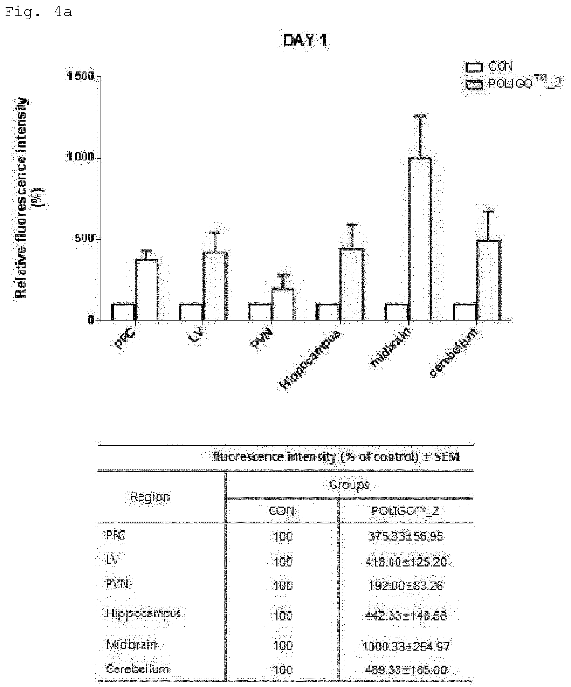

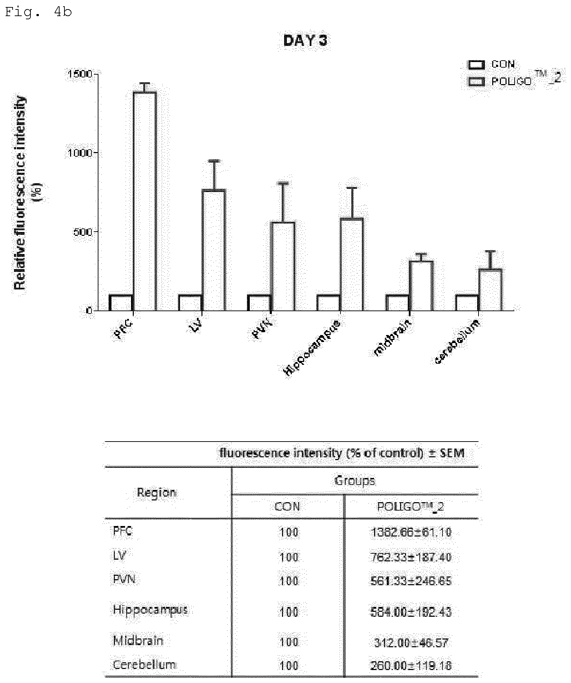

As a result, on day 1, distribution of fluorescence expression of the nucleic acid complex was determined to be high in the midbrain and cerebellum, which are the posterior regions of the brain, among various brain regions. When these results were numerically analyzed, the fluorescence value was found to have increased 2 or 3 times in the anterior region of the brain and a maximum of 10 times in the posterior region thereof in the group administered with the nucleic acid complex compared to the control group (FIG. 4a). On day 3, the distribution of fluorescence expression was determined to be high even in the prefrontal cortex, which is the anterior region of the brain, among the brain regions. When these results were numerically analyzed and compared, the fluorescence value was found to have increased at least 10 times in the anterior region of the brain and 2.5 times in the posterior region thereof in the group administered with the nucleic acid complex compared to the control group (FIG. 4b). By numerically analyzing the fluorescence intensity, the accuracy of the experimental results was improved.

### Industrial Applicability

The nucleic acid complex in which the bioactive nucleic acid and the carrier peptide nucleic acid modified to have an overall positive charge are complementarily bound to each other according to the present invention is capable of crossing the blood-brain barrier with excellent efficiency. In particular, when a drug is bound to the nucleic acid complex, the blood-brain barrier permeability of the drug is increased, so the present invention is very useful for the treatment or diagnosis of a disease.

Although specific embodiments of the present invention have been disclosed in detail above, it will be obvious to those of ordinary skill in the art that the description is merely of preferable exemplary embodiments, and is not to be construed as limiting the scope of the present invention. Therefore, the substantial scope of the present invention will be defined by the appended claims and equivalents thereof.

### Sequence List Free Text

An electronic file is attached.

## Claims

1. A composition for blood-brain barrier permeation comprising a nucleic acid complex having a structure of Structural Formula (1) below:
[Structural Formula (1)] [A ≡ C(+)]
wherein Structural Formula (1),
A represents a bioactive nucleic acid having a sequence capable of binding to a target gene or a target gene sequence,
C represents a carrier peptide nucleic acid capable of binding to the bioactive nucleic acid;
'≡' represents complementary binding between the bioactive nucleic acid and the carrier peptide nucleic acid,
the bioactive nucleic acid represented by A is a peptide nucleic acid and has an overall negative charge,
the carrier peptide nucleic acid represented by C(+) has an overall positive charge,
the nucleic acid complex of Structural Formula (1) represented by A ≡ C(+) has an overall positive charge, and
the carrier peptide nucleic acid comprises at least one gamma- or alpha-backbone-modified peptide nucleic acid monomer such that the carrier peptide nucleic acid has an overall positive charge, and the gamma- or alpha-backbone-modified peptide nucleic acid monomer comprises monomers having positively charged amino acids, a number of which is higher than a number of monomers having negatively charged amino acids, so the carrier peptide nucleic acid has an overall positive charge.

2. The composition according to claim 1, wherein the bioactive nucleic acid or the carrier peptide nucleic acid comprises a material for facilitating endosomal escape that is additionally bound to a 5'-end or a 3'-end of each nucleic acid.

3. The composition according to claim 2, wherein the material for facilitating endosomal escape is at least one selected from the group consisting of a peptide, lipid nanoparticles, polyplex nanoparticles, polymer nanospheres, inorganic nanoparticles, cationic lipid-based nanoparticles, a cationic polymer, and a pH-sensitive polymer.

4. The composition according to claim 3, wherein the peptide is GLFDIIKKIAESF (SEQ ID NO: 5) or histidine (10).

5. The composition according to claim 1, wherein the bioactive nucleic acid comprises a material for treating or diagnosing a disease that is additionally bound to a 5'-end or a 3'-end of the nucleic acid.

6. The composition according to claim 1, wherein each of the bioactive nucleic acid and the carrier peptide nucleic acid comprises 2 to 50 nucleic acid monomers.

7. The composition according to claim 1, wherein the carrier peptide nucleic acid comprises a sequence in which part or all of a nucleotide sequence is complementary to the bioactive nucleic acid.

8. The composition according to claim 7, wherein the carrier peptide nucleic acid comprising a sequence in which part of the nucleotide sequence is complementary comprises at least one universal base.

9. The composition according to claim 1, wherein the positively charged amino acid is at least one selected from the group consisting of lysine (Lys, K), arginine (Arg, R), histidine (His, H), diamino butyric acid (DAB), ornithine (Orn), and amino acid analogues.

10. The composition according to claim 1, wherein the negatively charged amino acid is glutamic acid (Glu, E), aspartic acid (Asp, D), or an amino acid analogue.

11. The composition according to claim 1, wherein the bioactive nucleic acid and the carrier peptide nucleic acid are bound through parallel binding or antiparallel binding in a 5' orientation and a 3' orientation of each nucleic acid.

12. The composition according to claim 1, wherein a binding affinity (melting temperature, Tm) between the bioactive nucleic acid and the carrier peptide nucleic acid is lower than a binding affinity between the bioactive nucleic acid and a gene targeted by the bioactive nucleic acid.

13. The composition according to claim 12, wherein the bioactive nucleic acid and the carrier peptide nucleic acid are bound through parallel binding or partial specific binding, so the binding affinity (melting temperature, Tm) between the bioactive nucleic acid and the carrier peptide nucleic acid is lower than the binding affinity between the bioactive nucleic acid and the gene targeted by the bioactive nucleic acid.

14. The composition according to claim 12, wherein the carrier peptide nucleic acid has at least one peptide nucleobase selected from among a linker, a universal base, and a peptide nucleobase having a base that is not complementary to a corresponding base of the bioactive nucleic acid, so the binding affinity (melting temperature, Tm) between the bioactive nucleic acid and the carrier peptide nucleic acid is lower than the binding affinity between the bioactive nucleic acid and the gene targeted by the bioactive nucleic acid.

15. The composition according to claim 12, wherein a time point of separation between the bioactive nucleic acid and the carrier peptide nucleic acid and a time point of binding between the bioactive nucleic acid and the gene targeted by the bioactive nucleic acid are able to be controlled by adjusting the binding affinity between the bioactive nucleic acid and the carrier peptide nucleic acid.

16. The composition according to claim 14, wherein the universal base is a base pairing with a natural base including adenine, guanine, cytosine, thymine, or uracil without selectivity and having binding affinity lower than complementary binding affinity, and is at least one selected from the group consisting of inosine PNA, indole PNA, nitroindole PNA, and abasic PNA.

17. The composition according to claim 1, wherein at least one material selected from the group consisting of a hydrophobic moiety, a hydrophilic moiety, a target antigen-specific antibody, an aptamer, a quencher, a fluorescent label, and a luminescent label is bound to the bioactive nucleic acid and/or the carrier peptide nucleic acid.

18. The composition according to claim 17, wherein the at least one material selected from the group consisting of a hydrophobic moiety, a hydrophilic moiety, a target antigen-specific antibody, an aptamer, a quencher, a fluorescent label, and a luminescent label is bound to the bioactive nucleic acid and/or the carrier peptide nucleic acid through simple covalent bonding or linker-mediated covalent bonding.

19. The composition according to claim 5, wherein the disease is a brain disease.

20. The composition according to claim 19, wherein the brain disease is selected from the group consisting of Alzheimer's disease, Parkinson's disease, Huntington's disease, Lou Gehrig's disease, Creutzfeldt-Jakob disease, Niemann-Pick disease, stroke, palsy, dementia, thrombosis, embolism, transient ischemic attack, small artery occlusion, intracerebral hemorrhage, cerebral infarction, head injury, cerebral circulatory metabolic disorder, brain functional coma, brain cancer, and brain tumor.

21. The composition according to claim 19, which is a pharmaceutical composition for preventing or treating the brain disease.

22. The composition according to claim 19, which is a composition for diagnosing the brain disease.
